# FASCICULE DE BREVET EUROPEEN

(11) **EP 2 854 770 B1**
(45) Date de publication et mention de la délivrance du brevet: **17.04.2019**
(21) Numéro de dépôt: 13727112.8
(22) Date de dépôt: 30.05.2013
(51) Int. Cl.: A61K 9/00, A61K 9/14, A61K 9/107, A61K 47/14, A61Q 19/00, A61K 8/36, A61K 8/37, A61K 8/39, A61K 8/81, A61K 8/06, A61K 8/49, A61K 9/127

(54) **PROCÉDÉ DE PRÉPARATION D'UNE COMPOSITION DERMATOLOGIQUE COMPRENANT DES OLÉOSOMES**
VERFAHREN ZUR HERSTELLUNG EINER DERMATOLOGISCHEN ZUSAMMENSETZUNG MIT OLEOSOMEN
METHOD FOR PREPARING A DERMATOLOGICAL COMPOSITION INCLUDING OLEOSOMES

(30) Priorité: 01.06.2012 FR 1255107; 01.06.2012 US 201261654680 P
(43) Date de publication de la demande: 08.04.2015
(73) Titulaire: Galderma Research & Development, 06410 Biot (FR)
(72) Inventeur: DJEDOUR, Amel Safia, 06600 Antibes (FR)
(74) Mandataire: Casalonga
(86) Numéro de dépôt international: PCT/EP2013/061192
(87) Numéro de publication internationale: WO 2013/178752

(56) Documents cités:
- EP-A1- 0 641 557
- EP-A1- 0 973 484
- WO-A1-2005/079730

## Description

La présente invention se rapporte à un procédé de fabrication d'une composition pharmaceutique, notamment dermatologique, ou cosmétique comprenant des oléosomes dispersés dans une phase aqueuse.

Les compositions de type émulsion huile-dans-eau comprenant des globules liquides enrobés, également appelés oléosomes, sont connues notamment du brevet EP 0 641 557. Il s'agit d'émulsions de type huile-dans-eau, formées de globules liquides enrobés (oléosomes), pourvus chacun d'un enrobage cristal liquide lamellaire et dispersés dans une phase aqueuse.

Ces oléosomes permettent aussi bien l'encapsulation d'un principe actif lipophile dans les globules liquides, que la solubilisation ou la dispersion du principe actif dans la phase aqueuse externe.

Le procédé d'obtention de ces systèmes décrit dans l'art antérieur est particulier et comprend :
- le chauffage des deux phases (grasse et aqueuse) à une température de 80 à 95°C ;
- la pré-émulsification des deux phases chauffées à l'aide d'un homogénéisateur pendant 30 minutes ;
- plusieurs passages successifs dans un Homogénéisateur Haute Pression (HHP) à 500 Bars ;
- le refroidissement de l'émulsion pendant environ 60 minutes ; et enfin
- l'addition de la phase aqueuse finale (par exemple un gel) et l'agitation à l'aide d'une turbine équipée d'une pale défloculeuse.

Or, un tel procédé particulier d'obtention de ces systèmes présente de nombreux inconvénients :
- il est complexe, long, met en oeuvre de nombreuses étapes (chauffage, pré-émulsification, plusieurs passages successifs au HHP, refroidissement, gélification, ce qui fait une moyenne de 7 étapes) et utilise des équipements différents pour chacune ;
- il nécessite un équipement particulier, i.e. l'Homogénéisateur Haute Pression (HHP), pour la formation des oléosomes au moment de l'émulsification ;
- les températures de chauffage sont élevées (80 à 95°C) avant le mélange des deux phases, ce qui exclut l'incorporation d'excipients et d'actifs sensibles à la chaleur.

De plus, les passages successifs du mélange au HHP sous pression entraînent une augmentation supplémentaire de la température du mélange, qu'il n'est pas possible de contrôler ; le procédé peut donc entraîner la dégradation du ou des principes actifs sensibles à la chaleur pouvant être introduits dans la composition. Cela constitue donc un facteur limitant vis-à-vis des principes actifs et excipients utilisables.

Il existe donc un besoin pour un procédé de fabrication d'oléosomes simple à mettre en oeuvre, facilement industrialisable et qui utilise les équipements de production conventionnels.

Le problème que se propose de résoudre ici la présente invention, est donc de concevoir un procédé de fabrication d'une composition stable comprenant des oléosomes, qui soit simple, facilement industrialisable, qui utilise des équipements conventionnels, qui mette en oeuvre des températures inférieures à 75°C et qui soit ainsi adapté à l'incorporation d'excipients et / ou de principes actifs sensibles à la chaleur.

La Demanderesse a donc, de façon surprenante, découvert qu'un procédé spécifique, n'utilisant pas d'HHP, permet l'obtention d'oléosomes stables.

La présente invention a donc pour objet un procédé de fabrication d'une composition pharmaceutique ou cosmétique se trouvant sous la forme d'une émulsion de type huile-dans-eau formée de globules liquides et pourvus chacun d'un enrobage cristal liquide lamellaire et dispersés dans une phase aqueuse, chaque globule liquide étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct,
la composition étant apte à incorporer des excipients et/ou des principes actifs thermosensible à des températures supérieures à 75°C,
ledit procédé comprenant les étapes suivantes :
a) mélanger, à une température d'au plus 75°C, de préférence comprise entre 65°C et 75°C, l'agent tensioactif lipophile, l'agent tensioactif hydrophile, le tensioactif anionique distinct et tous les ingrédients des globules liquides, afin d'obtenir un mélange homogène de phase grasse (A),
b) mélanger, à une température d'au plus 75°C, de préférence comprise entre 65°C et 75°C, tous les ingrédients de la phase aqueuse, afin d'obtenir un mélange homogène de phase aqueuse (B),
c) verser la phase aqueuse (B) obtenue en b) sur la phase grasse (A) obtenue en a) soit à l'aide d'un agitateur rotor-stator à une vitesse comprise entre 10000 rpm et 13000 rpm et pendant un temps compris entre 25 minutes et 1 heure, soit à l'aide d'une turbine dont la vitesse de rotation est supérieure à 700 rpm, afin d'obtenir une émulsion homogène,
d) refroidir l'émulsion homogène obtenue en c), sous agitation à une vitesse comprise entre 1000 rpm et 3000 rpm, et à une vitesse coaxiale comprise entre 40 rpm et 50 rpm, jusqu'à ce que la température de l'émulsion soit inférieure ou égale à 50°C,
ledit procédé comportant un nombre d'étapes inférieur ou égal à 5.

Le procédé ne nécessite pas l'utilisation d'un homogénéisateur haute pression, ne présente pas d'étape de pré-émulsification et tout étape dudit procédé est effectuée à une température inférieure ou égale à 75°C.

Comme détaillé dans les exemples, le procédé selon l'invention est facilement industrialisable. En effet, différentes tailles de lot ont été réalisées (laboratoire, pilote et industriel), ce qui démontre que le procédé selon l'invention est à la fois robuste, reproductible et transposable aux différents équipements conventionnels utilisés habituellement, tels qu'un agitateur rotor-stator, une turbine d'agitation, une pale défloculeuse, et un agitateur planétaire (râcleurs).

Le procédé selon l'invention présente ainsi les avantages suivants :
- il nécessite peu d'étapes de fabrication, environ 4 étapes. Il est donc plus rapide et plus facile à mettre en oeuvre que les procédés de l'art antérieur, spécialement lors de la production industrielle ;
- il ne nécessite pas d'étape de pré-émulsification,
- il ne nécessite pas d'équipements particuliers, difficiles à transposer à l'échelle industrielle. La fabrication de lots de tailles différentes se fait grâce à l'utilisation d'équipements conventionnels classiquement utilisés dans l'industrie pharmaceutique ou cosmétique ;
- les températures de chauffage avant émulsification des deux phases sont d'au maximum 70°C à 75°C. Cette température permet ainsi l'incorporation dans la composition d'excipients et/ou de principes actifs sensibles à la chaleur ;
ni la mise en oeuvre de forts cisaillements sous pression, ce qui limite donc les risques de déstabilisation ou de dégradation de la composition.

Typiquement, le procédé selon l'invention présente donc les caractéristiques suivantes :
- Il présente un nombre d'étape(s) inférieur ou égal à 5,
- Il utilise des températures de mélange d'au plus 75°C,
- Il ne nécessite pas d'étape de pré-émulsification,
- Il ne nécessite pas l'utilisation d'homogénéisateur haute pression. De préférence donc, il ne comprend aucune étape d'homogénéisation haute pression.

Ainsi, le procédé selon l'invention limite les risques de déstabilisation ou de dégradation de la composition, i.e. il permet l'obtention d'une composition stable physiquement et chimiquement à 3 mois à température ambiante. Selon un mode de réalisation particulier, l'invention vise l'utilisation du procédé pour la fabrication d'une composition comprenant au moins un excipient et/ou un principe actif thermosensible, c'est-à-dire se dégradant à des températures supérieures à 75°C.

En outre, le procédé selon l'invention permet d'obtenir des compositions pour lesquelles le rapport entre la phase aqueuse et la phase grasse (globules liquides enrobés) est le suivant: % Phase grasse / (% phase grasse + % phase aqueuse) compris entre 0.40 et 0.55, de préférence comprise entre 0.45 et 0.50, de préférence égal à 0.48.

### Composition obtenue par le procédé selon l'invention

La composition obtenue par le procédé selon l'invention peut être une composition pharmaceutique, notamment dermatologique ou une composition cosmétique C'est une émulsion huile-dans-eau, formée d'oléosomes dispersés dans une phase aqueuse.

Par oléosomes, on entend les globules liquides unitairement enrobés d'une couche cristal liquide monolamellaire ou oligolamellaire obtenue à partir d'au moins un agent tensioactif lipophile, d'au moins un agent tensioactif hydrophile et d'au moins un tensioactif anionique distinct. Ces oléosomes sont dispersés dans la phase aqueuse (phase continue).

Les oléosomes sont à distinguer des nanosphères lipidiques. En effet, les oléosomes sont des globules liquides enrobés par une enveloppe non polymérique solide à température ambiante (i.e. formée à partir d'au moins un agent tensioactif lipophile, d'au moins un agent tensioactif hydrophile et d'au moins un tensioactif anionique distinct), par opposition à des nanosphères lipidiques qui sont des particules matricielles, i.e. dont la totalité de la masse est solide à température ambiante.

Les oléosomes (globules liquides enrobés) présentent un diamètre moyen inférieur à 800nm, de préférence inférieur à 600nm et plus particulièrement inférieur à 500nm. Le diamètre moyen des oléosomes et la distribution granulométrique peuvent être déterminés par DLS (Dynamic Light Scattering) au moyen d'un granulomètre de type Zetasizer Nano ZS (Malvern Instruments), comme cela est expliqué dans les exemples.

La composition peut en outre comprendre au moins un principe actif, qui peut être soit solubilisé dans les globules liquides, soit solubilisé dans la phase aqueuse continue, soit dispersé au sein de la composition. Par couche oligolamellaire, on entend une couche comprenant de 2 à 5 lamelles lipidiques.

La composition obtenue par le procédé selon l'invention est stable physiquement et chimiquement.

Par stabilité physique selon l'invention, on entend une composition dont les propriétés physiques, telles que les caractères organoleptiques, pH et viscosité, sont stables au cours du temps et à différentes conditions de température (par exemple 4°C, température ambiante et 40°C).

Par température ambiante, on entend une température comprise entre 15 et 25°C.

Par stabilité chimique selon l'invention, on entend une composition dans laquelle l'actif pharmaceutique ou cosmétique est stable chimiquement au cours du temps et ce quelle que soit la condition de température : 4°C, température ambiante, 40°C.

De préférence, l'agent tensioactif lipophile utilisé pour la formation des oléosomes présente une HLB comprise entre 2 et 5.

A titre d'agent tensioactif lipophile présentant une HLB comprise entre 2 et 5 utilisable selon l'invention, on citera les esters de sorbitane, tels que le monostéarate de sorbitane (HLB = 4.7) vendu sous le nom de Span® 60 par la société Croda, les esters de glycérol tel que le monostéarate de glycerol vendu sous le nom de Kolliwax® GMS II (HLB=3.8) par la société BASF, les sucroesters de basse HLB tel que le sucrose dilaurate vendu sous le nom de Surfhope® C-1205 (HLB=5) ou le sucrose tristéarate vendu sous le nom de Surfhope® C-1803 (HLB=3) par la société Gattefossé, ou encore le stéaryl éther polyoxyéthyléné comportant 2 motifs oxyéthylène (2 OE).

De préférence, l'agent tensioactif lipophile présentant une HLB comprise entre 2 et 5 est choisi parmi le distéarate de sucrose, le tristéarate de sucrose, le distéarate de diglycéryle, le tristéarate de tétraglycéryle, le décastéarate de décaglycéryle, le monostéarate de diglycérol, le monostéarate de sorbitane, le tristéarate de sorbitane, le monostéarate de 15 diéthylène glycol, l'ester de glycérol et d'acides palmitique et stéarique, le monostéarate polyoxyéthyléné comportant 2 motifs oxyéthylène (2 OE), le stéaryl éther polyoxyéthyléné comportant 2 motifs oxyéthylène (2 OE), le mono- et dibéhénate de glycéryle et le tétrastéarate de pentaérythritol.

De préférence, l'agent tensioactif hydrophile utilisé pour la formation des oléosomes présente une HLB comprise entre 8 et 12.

On peut citer, à titre d'exemple non limitatif d'agent tensioactif hydrophile présentant une HLB entre 8 et 12, les esters de polyoxyéthylène glycol tel que le glycéryl stéarate and PEG 100 stéarate vendu sous le nom de Arlacel® 165 FL (HLB=11) par la société Croda , le PEG 6 Stéarate et PEG 32 stéarate vendu sous le nom de Tefose® 1500 (HLB= 10) par la société Gateffossé, les esters de sorbitane polyoxyéthyléné et les sucroesters de haut HLB tel que le sucrose stéarate vendu sous le nom de Surfhope® C-1811 (HLB=11) par la société Gattefossé, ou encore le stéaryl éther polyoxyéthyléné comportant 10 motifs oxyéthylène. De préférence, l'agent tensioactif hydrophile présentant une HLB comprise entre 8 et 12 est choisi parmi le monostéarate de sorbitane polyoxyéthyléné 4 OE (POE-4 sorbitan monostéarate ou polysorbate-61), le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le stéaryl éther polyoxyéthyléné 10 OE, le distéarate polyoxyéthyléné 12 OE et le distéarate de méthylglucose polyoxyéthyléné 20 OE.

Par tensioactif anionique distinct, on entend un tensioactif anionique différent de l'agent tensioactif lipophile et de l'agent tensioactif hydrophile formant l'enrobage des oléosomes. De préférence, le tensioactif anionique distinct est un acide gras ou un ester d'acide gras, ledit acide gras comprenant au moins une chaîne grasse saturée ayant plus de 12 atomes de carbone, de préférence entre 16 et 22.

Le tensioactif anionique distinct peut également être un lipide amphiphile ionique. Le lipide amphiphile ionique est de préférence choisi parmi le groupe comprenant les lipides anioniques neutralisés, les lipides amphotères et les dérivés alkylsulfoniques.

Les lipides anioniques neutralisés sont choisis en particulier parmi :
- les sels alcalins du dicétylphosphate, et en particulier les sels de sodium et de potassium ;
- les sels alcalins du dimyristylphosphate, et en particulier les sels de sodium et de potassium ;
- les sels alcalins du cholestérol sulfate, et en particulier le sel de sodium ;
- les sels alcalins du cholestérol phosphate, et en particulier le sel de sodium ;
- les sels monosodique et disodique des acides acylglutamiques, et en particulier les sels monosodique et disodique de l'acide N-stéaroylglutamique, le sel de sodium de l'acide phosphatidique.

Les lipides amphotères sont choisis en particulier parmi les phospholipides et notamment la phosphatidyléthanolamine de soja pure.

Les dérivés alkylsulfoniques sont avantageusement les composés de formule : 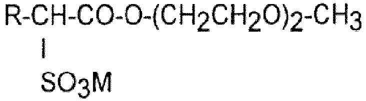 dans laquelle R représente les radicaux C₁₆H₃₃ et C₁₈H₃₇ pris en mélange ou séparément et M est un métal alcalin, de préférence le sodium.

Plus préférentiellement, le tensioactif anionique distinct est choisi parmi l'acide stéarique, l'acide palmitique, l'acide arachidique et l'acide béhénique. Il peut également être le stéaroyl glutamate de sodium, commercialisé sous la dénomination Eumulgin® SG par BASF ou Amisoft® HS 11 par Ajinomoto.

De préférence, les tensioactifs sont mélangés dans les proportions suivantes pour former les oléosomes :
o Tensioactif lipophile (HLB comprise entre 2 et 5) : 45-50% en poids du mélange ;
o Tensioactif hydrophile (HLB comprise entre 8 et 12) : 30-35% en poids du mélange ; et
o Tensioactif anionique distinct : 20-25% en poids du mélange.

De préférence, la quantité totale de tensioactifs présents dans une composition obtenue par le procédé selon l'invention est comprise entre 3 et 4% en poids total de la composition.

Les globules liquides sont typiquement composés d'au moins un excipient huileux choisi parmi les esters de tocophérols, les acides gras polyéthoxylés, les triglycérides et huiles en contenant et les esters d'acides gras. Ils peuvent également comprendre un ou plusieurs corps gras liquides ou semi-liquides à température ambiante. Ces composés sont notamment des huiles minérales, des huiles végétales, des huiles animales ou des huiles de silicone.

Par phase huileuse ou excipient huileux, on entend toute matière non miscible à l'eau d'origine, naturelle, animale ou synthétique.

Parmi les triglycérides et huiles en contenant, on peut citer à titre non limitatif, les triglycérides d'acide octanoïque ou encore les triglycérides des acides caprylique/caprique comme ceux vendus par la société Stearineries Dubois ou ceux vendus sous la dénomination Miglyol® 810, 812, et 818 par la société Sasol.

Parmi les esters d'acide gras on peut citer à titre non limitatif le diisopropyl adipate, le cétostéaryl isononanoate.

Comme exemple d'huile minérale, on peut citer par exemple des huiles de paraffine de différentes viscosités telles que le Primol® 352, le Marcol® 52, Marcol® 152 vendus par la société Esso.

Comme huile végétale, on peut citer l'huile d'amande douce, l'huile de coprah, l'huile de palme, l'huile de soja, l'huile de sésame, l'huile de tournesol.

Comme huile animale, on peut citer la lanoline, le squalène, l'huile de poisson, l'huile de vison avec comme dérivé le squalane vendu sous le nom Cosbiol® par la société Laserson.

Comme huile de silicone volatile ou non volatile, on peut citer des diméthicones comme les produits vendus sous le nom de Q7-9120® Silicone Fluid de viscosité 20 cSt à 12500 cSt ou le produit vendu sous le nom ST-Cyclomethicone-5 NF® par la société Dow Corning.

De préférence, la composition selon l'invention présente un rapport entre la quantité totale de tensioactifs et la quantité totale de phase huileuse constituant les globules liquides compris entre 10% et 25%. Avec un tel rapport, les oléosomes présentent une taille acceptable, avec un enrobage cristal liquide lamellaire autour des gouttelettes d'huile mono- ou oligo-lamellaire.

Les oléosomes sont dispersés dans une phase aqueuse, qui est la phase continue.

La phase aqueuse continue comprend de l'eau. Cette eau peut être de l'eau déminéralisée, une eau florale telle que l'eau de bleuet, ou une eau thermale ou minérale naturelle, par exemple choisie parmi l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau de Néris-les-Bains, l'eau d'Allevard-les-Bains, l'eau de Digne, l'eau de Maizières, l'eau de Neyrac-les-Bains, l'eau de Lons-le-Saunier, les Eaux Bonnes, l'eau de Rochefort, l'eau de Saint Christau, l'eau des Fumades et l'eau de Tercis-les-bains, l'eau d'Avène ou l'eau d'Aix les Bains.

L'eau peut être présente à une teneur comprise entre 25 et 90 % en poids par rapport au poids total de la composition, de préférence comprise entre 50 et 90 % en poids.

La phase aqueuse peut comprendre également des agents humectants, préférentiellement des polyols sélectionnés parmi la glycérine, le propylène glycol, le dipropylène glycol, le pentylène glycol, la diglycérine ou le sorbitol.

La phase aqueuse peut comprendre également à l'état dissous un agent basique. Cet agent basique permet la neutralisation de l'acide gras présent dans les oléosomes en tant que tensioactif anionique distinct. Cet agent basique peut être la triéthanolamine, la soude, la lysine ou bien encore l'arginine.

La phase aqueuse peut également comprendre de manière optionnelle des conservateurs, utilisés seul en association afin de protéger efficacement les formules contre toute contamination bactérienne. Ces conservateurs utilisés préférentiellement dans l'invention sont l'acide benzoïque et le sorbate de potassium. Ils peuvent être utilisés à une taux de 0.01 à 5% et préférentiellement de 0.01 à 2%.

La phase aqueuse peut également comprendre de manière optionnelle des additifs parmi lesquels on peut citer les catégories suivantes seules ou en combinaison : des agents chélatants, dans anti-oxydants, des agents apaisants et/ou anti-irritants, ou tout autre additif usuellement utilisé dans le domaine pharmaceutique et cosmétique permettant de conférer à la dite préparation des propriétés spécifiques.

La composition obtenue par le procédé selon l'invention peut comprendre au moins un actif pharmaceutique ou cosmétique. Ce principe actif peut être choisi parmi les rétinoïdes, l'acide glycyrrhétinique et le gluconate de zinc, seul ou en mélange.

Encore plus préférentiellement, le principe actif est choisi parmi les rétinoïdes.

Les rétinoïdes pouvant être utilisés dans le cadre de l'invention comprennent notamment l'acide tout-trans rétinoïque ou trétinoïne, l'acide 13-cis-rétinoïque ou isotrétinoïne, l'acitrétine, l'acide arotinoïque, le rétinol, l'adapalène, le tazarotène, le rétinaldéhyde, l'étrétinate et, les composés protégés dans la demande de brevet WO2006/066978 tel que l'acide 3"-tert-butyl-4'-(2-hydroxy-ethoxy)-4"-pyrrolidin-1-yl-[1,1';3',1"]-terphenyl-4-carboxylique, les composés de la demande de brevet FR0512367 dont l'acide 2-hydroxy-4-[3-hydroxy-3-(5,6,7,8-tétrahydro-5,5,8,8-tétraméthyl-2-naphtyl)-1-propynyl]benzoique ou l'un de ses énantiomères, les composés de la demande de brevet WO 05/56516 dont l'acide 4'-(4-isopropylamino-butoxy)-3'-(5,5,8,8-tetramethyl-5,6,7,8-tetrahydro-naphthalen-2-yl)-biphenyl-4-carboxylique, les composés de la demande de brevet PCT/EP04/014809 dont l'acide 4-{3-hydroxy-3-[4-(2-éthoxy-ethoxy)-5,5,8,8-tetramethyl-5,6,7,8-tetrahydro-naphthalen-2-yl]-prop-1-ynyl}-benzoïque, les composés de la demande de brevet FR 2 861 069 dont l'acide 4-[2-(3-tert-butyl-4-diethylamino-phenyl)-2-hydroxyimino-ethoxy]-2-hydroxy-benzoïque. L'acide 3"-tert-butyl-4'-(2-hydroxy-ethoxy)-4"-pyrrolidin-1-yl-[1,1';3',1"]-terphenyl-4-carboxylique tel que protégé dans la demande WO2006/066978 est particulièrement préféré.

Dans le cas où le principe actif est de type rétinoïde, le principe actif peut être présent en quantité comprise entre 0.00001 et 0.3% en poids par rapport au poids total de la composition, de préférence de 0.0001 à 0.1% et de manière préférentielle, de 0.001% à 0.05% en poids par rapport au poids total de la composition. Dans un mode préféré, la composition obtenue selon le procédé de l'invention, comprend entre 0.001 et 0.05% d'acide 3"-tert-butyl-4'-(2-hydroxy-ethoxy)-4"-pyrrolidin-1-yl-[1,1';3',1"]-terphenyl-4-carboxylique, plus particulièrement entre 0.003 et 0.03 % en poids par rapport au poids total de la composition.

Dans un autre mode selon l'invention, l'homme de l'art définira les gammes de concentration en fonction de la nature des actifs et l'adaptera selon ses connaissances à l'utilisation préférée de la composition selon l'invention.

La composition obtenue par le procédé selon l'invention selon l'invention peut être incorporée dans un véhicule pharmaceutiquement ou cosmétiquement acceptable, tel un gel, une solution ou une émulsion comme une crème ou une lotion, pouvant être pulvérisable ou non.

Lorsque le véhicule pharmaceutiquement ou cosmétiquement acceptable est un gel, la composition selon l'invention est dispersée dans une phase hydrophile qui comprend au moins un agent gélifiant. Cet agent gélifiant peut être un dérivé cellulosique choisi parmi les gélifiants cellulosiques semi-synthétiques, tels que la méthylcellulose, l'éthylcellulose, l'hydroxypropylméthylcellulose commercialisés par la société Colorcon sous le nom de Methocel® (par exemple : Methocel® E4M), l'hydroxyéthylcellulose commercialisés par la société Ashland sous le nom de Natrosol® (par exemple : Natrosol® 250 HHX), la carboxyméthylcellulose, l'hydroxyméthylcellulose, et l'hydroxypropylcellulose, pris seuls ou en mélange.. L'agent gélifiant peut également être choisi parmi les gommes naturelles comme la gomme de tragacanthe, la gomme de guar, la gomme d'acacia, la gomme arabique, la gomme xanthane, l'amidon et ses dérivés, les copolymères d'acide polyacrylique comme par exemple les carbomers commercialisés par la société Lubrizol (i.e. Carbomer® 980) et de methylmethacrylate, les carboxyvinyl polymères, les polyvinyl pyrrolidones et leurs dérivés, les polyvinyl alcools, les biopolymères tels que l'alginate de sodium, la pectine, la dextrine, le chitosan, le hyaluronate de sodium et leurs dérivés pris seuls ou en mélange, L'agent gélifiant peut également être choisi parmi le composé Sepigel® 305 constitué d'un mélange polyacrylamide / isoparaffine en C13-C14/ laureth-7, ou le Simulgel® 600PHA ou Sepineo® P600, à savoir le sodium acryloyldiméthyltaurate copolymère/ isohexadecane/polysorbate 80, ces deux produits étant commercialisés par la société Seppic.

L'agent gélifiant est utilisé notamment à une concentration comprise entre 0,1 et 10% en poids, de préférence entre 0,1 et 4% en poids par rapport au poids total de la composition.

Lorsque le véhicule pharmaceutiquement ou cosmétiquement acceptable est une solution, la composition selon l'invention est dispersée au sein d'un véhicule composé d'une phase aqueuse (telle que définie précédemment dans la présente invention).

Lorsque le véhicule pharmaceutiquement ou cosmétiquement acceptable est une crème ou une lotion, la composition selon l'invention est dispersée au sein d'un véhicule composé d'une phase aqueuse et d'une phase grasse comprenant ou non au moins un surfactant ou émulsionnant.

La composition selon l'invention pourra en outre contenir des additifs ou combinaisons d'additifs, tels que :
- des agents co-tensioactifs comme les alcools gras ;
- des agents conservateurs;
- des agents propénétrants ;
- des agents stabilisants ;
- des agents humectants ;
- des agents propénétrants ;
- des agents stabilisants ;
- des agents humectants ;
- des agents régulateurs d'humidité ;
- des agents régulateurs de pH ;
- des agents modificateurs de pression osmotique ;
- des agents chélatants ;
- des filtres UV-A et UV-B ;
- et des antioxydants.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée. Ces additifs peuvent être présents dans la composition de 0 à 40% en poids par rapport au poids total de la composition.

Lorsque la composition obtenue selon le procédé de l'invention comprend un actif pharmaceutique, la composition est utilisable comme médicament.

En particulier, lorsque la composition obtenue selon le procédé de l'invention contient un rétinoide, elle peut être utilisée pour traiter les affections dermatologiques, notamment humaines, en particulier l'acné, le psoriasis, la dermatite atopique ou encore la kératose palmo-plantaire.

Lorsque la composition obtenue selon le procédé de l'invention ne comprend pas de principe actif pharmaceutique, ou comprend un actif cosmétique, la composition est utilisable en cosmétique dans le domaine des soins de la peau, des phanères, du corps ou des cheveux. La composition présente notamment un avantage dans le domaine de la protection solaire. En effet, il a été noté que les compositions contenant des oléosomes présentent des indices de protection solaire améliorés par rapport à des compositions ne contenant pas ces oléosomes. Les compositions obtenues selon le procédé de l'invention présentent également cet avantage.

### Procédé selon l'invention

Comme indiqué ci-avant, la présente invention a pour objet un procédé de fabrication d'une composition pharmaceutique ou cosmétique se trouvant sous la forme d'une émulsion de type huile-dans-eau formée de globules liquides pourvus chacun d'un enrobage cristal liquide lamellaire et dispersés dans une phase aqueuse, chaque globule liquide étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct, comprenant les étapes suivantes :
a) mélanger, à une température d'au plus 75°C, de préférence comprise entre 65°C et 75°C, l'agent tensioactif lipophile, l'agent tensioactif hydrophile, le tensioactif anionique distinct et tous les ingrédients des globules liquides, afin d'obtenir un mélange homogène de phase grasse (A),
b) mélanger, à une température d'au plus 75°C, de préférence comprise entre 65°C et 75°C, tous les ingrédients de la phase aqueuse, afin d'obtenir un mélange homogène de phase aqueuse (B),
c) verser la phase aqueuse (B) obtenue en b) sur la phase grasse (A) obtenue en a) sous agitation forte, afin d'obtenir une émulsion homogène,
d) refroidir l'émulsion homogène obtenue en c), sous agitation lente, jusqu'à ce que la température de l'émulsion soit inférieure ou égale à 50°C.

Ce procédé est simple et n'utilise pas d'homogénéisateur haute pression (HHP).

Par ailleurs, le procédé selon l'invention ne nécessite pas d'étape de pré-émulsification et toute étape dudit procédé est effectuée à une température inférieure ou égale à 75°C.

Par agitation forte, on entend une agitation réalisée :
- au moyen d'un homogénéisateur de type rotor/stator dont la vitesse de rotation est comprise entre 10000 rpm et 13000rpm
- au moyen d'une turbine, dont la vitesse de rotation est supérieure à 700 rpm et préférentiellement supérieur à 1000 rpm, plus préférentiellement supérieure à 2000 rpm

Les mélanges des étapes a) et b) peuvent être concomitants ou successifs.

De préférence, le mélange de l'étape a) se fait à l'aide d'une turbine d'agitation, de préférence à une vitesse comprise entre 700 rpm et 1500 rpm, et éventuellement avec une vitesse coaxiale comprise entre 20 rpm et 40 rpm. De préférence, ce mélange se fait à une température de 70°C environ. A l'issue de cette étape a), on obtient la phase grasse (A). De préférence, le mélange de l'étape b) se fait à l'aide d'une turbine d'agitation, de préférence à une vitesse comprise entre 150 rpm et 1500 rpm. De préférence, ce mélange se fait à une température de 70°C environ. A l'issue de cette étape b), on obtient la phase aqueuse (B). Cette phase aqueuse comprend de l'eau, et éventuellement des composés hydrophiles, notamment un agent basique.

Ensuite, la phase aqueuse (B) est versée sur la phase grasse (A) sous agitation forte (étape c)): c'est l'étape d'émulsification.

Lorsque le lot est fabriqué à l'échelle laboratoire, la phase aqueuse (B) est versée sur la phase grasse (A) à l'aide d'un agitateur rotor-stator à une vitesse comprise entre 10000 rpm et 13000 rpm, et pendant un temps compris entre 25 minutes et 1 heure. De préférence, l'émulsification selon l'étape c) se fait pendant un temps compris entre 30 et 40 minutes.

Lorsque le lot est fabriqué à l'échelle pilote ou industrielle, l'émulsification se fait par aspiration de la phase aqueuse (B) dans la phase grasse (A), à une vitesse coaxiale comprise entre 45 rpm et 100 rpm, et sous agitation à une vitesse comprise entre 1000 rpm et 3000 rpm. Par exemple, l'émulsification peut être réalisée par aspiration de la phase aqueuse (B) dans la phase grasse (A) avec un tuyau, à une vitesse coaxiale comprise entre 45 rpm et 100 rpm, et sous agitation à une vitesse comprise entre 1000 rpm et 3000 rpm, et éventuellement avec une vitesse de raclage de 25 rpm environ.

Dans l'étape c), typiquement, l'agitation est maintenue jusqu'à l'obtention d'un liquide présentant un tapis d'émulsion très fin au microscope optique.

Puis, le mélange obtenu à l'étape c) est refroidi : c'est l'étape de refroidissement d). Typiquement, le refroidissement de l'étape d) se fait par l'introduction d'eau dans le mélange obtenu en c), sous agitation à une vitesse comprise entre 1000 rpm et 3000 rpm, et à une vitesse coaxiale comprise entre 40 rpm et 50 rpm, jusqu'à ce que la température de l'émulsion soit inférieure ou égale à 50°C.

De préférence, le refroidissement de l'étape d) se fait par l'introduction d'eau dans le mélange obtenu en c), sous agitation à une vitesse comprise entre 1000 rpm et 3000 rpm, et à une vitesse coaxiale comprise entre 40 rpm et 50 rpm, jusqu'à ce que la température de l'émulsion soit inférieure ou égale à 40°C.

En fin d'étape d), une émulsion comprenant les oléosomes est obtenue.

Cette émulsion peut être utilisée telle quelle, ou bien gélifiée. Dans ce dernier cas, un agent gélifiant peut être introduit dans le mélange refroidi obtenu en d), sous agitation à une vitesse comprise entre 1000 rpm et 2000 rpm, jusqu'à obtention d'une crème.

Lorsque l'émulsion comprend un principe actif, ce dernier peut être introduit dans la phase grasse lors de l'étape a), ou dans la phase aqueuse (B) lors de l'étape b), ou bien après l'étape c) d'émulsification, ou encore après l'étape d) de refroidissement.

Il va être maintenant donné, à titre d'illustration et sans aucun caractère limitatif, divers procédés de fabrication de formulations selon l'invention.

### Exemple 1: Procédé de fabrication à l'échelle laboratoire (100 g - 2 kg)

La composition qui suit est préparée selon le protocole indiqué ci-après.

Ses aspects macroscopique et microscopique à T0 sont étudiés, et le pH est mesuré.

La viscosité est également mesurée à l'aide d'un appareil de type Brookfield.

Les stabilités physique et chimique sont évaluées à 1, 2 et 3 mois, à température ambiante (TA) et à 40°C.

La taille des olésosomes a été vérifiée juste après la fabrication par DLS (Dynamic Light Scattering) au moyen d'un granulomètre de type Zetasizer Nano ZS (Malvern Instruments), après dilution des échantillons dans de l'eau distillée.

Les résultats obtenus figurent à la suite de chaque composition.

| | **EXEMPLE 1 - Composition** | | | | | |
|---|---|---|---|---|---|---|
| | **Nom Commercial** | **Nom INCI** | | | **%** | |
| | **-** | **Acide 3"-tert-butyl-4'-(2-hydroxy-ethoxy)-4"-pyrrolidin-1-yl-[1,1';3',1"]-terphenyl-4-carboxylique (ci-après "actif")** | | | **0.01** | |
| **A** | **BRIJ S10-SO** | **STEARETH-10** | | | **1.25** | |
| **A** | **SPEZIOL L2SM GS PHARMA** | **STEARIC-/ PALMITIC ACID** | | | **0.85** | |
| A | **BRIJ S2-SO** | **STEARETH-2** | | | **1.80** | |
| A | **SPEZIOL C18 PHARMA** | **STEARYL ALCOHOL** | | | **0.25** | |
| A | **MIGLYOL 812 N** | **CAPRYLIC/ CAPRIC TRIGLYCÉRIDES** | | | **15.50** | |
| A | **PHENOXETOL** | **PHENOXYETHANOL** | | | **1.00** | |
| A | **DL-ALPHA-TOCOPHÉROL** | **ALPHA TOCOPHEROL** | | | **0.20** | |
| A | **ST-CYCLOMETHICONE 5-NF** | **CYCLOPENTASILOXANE** | | | **4.00** | |
| **B** | **EAU PURIFIÉE** | **AQUA (WATER)** | | | **21.80** | |
| B | **TRIÉTHANOLAMINE** | **TRIETHANOLAMINE** | | | **0.20** | |
| B | **TITRIPLEX III** | **DISODIUM EDETATE** | | | **0.10** | |
| B | **GLYCÉRINE** | **GLYCEROL** | | | **5.00** | |
| B | **NIPAGIN M** | **METHYL PARABEN** | | | **0.20** | |
| **C** | **EAU PURIFIÉE** | **AQUA (WATER)** | | | **43.84** | |
| **D** | **SIMULGEL 600 PHA** | **ACRYLAMIDE / SODIUM ACRYLOYLDIMETHYL TAURATE COPOLYMER & ISOHEXADECANE & POLYSORBATE 80** | | | **4.00** | |
| | **CARACTERISATION À T0** | **ASPECT MACROSCOPIQUE** | | | **Crème épaisse blanche brillante** | |
| | | **ASPECT MICROSCOPIQUE** | | | **Tapis d'émulsion fin et homogène** | |
| | | **pH** | | | **7.53** | |
| | | **VISCOSITE** | | | **830000cp** | |
| | | | | | **92.8%** | |
| | | **DIAMETRE MOYEN** | | | **377 nm** | |
| | | | | | | |

| | **Suivi des stabilités** | **1MOIS** | | | **2MOIS** | **3MOIS** |
|---|---|---|---|---|---|---|
| | **Stabilité physique** | **pH TA/ 40°C** | **7.45/7.37** | | **7.38/7.29** | **7.42/7.27** |
| | | **Viscosité** | **TA** | **827000cP** | **831000 cP** | **826500 cP** |
| | | | | **91.4%** | **89.8%** | **83.2%** |
| | | | **40°C** | **826000 cP** | **821000 cP** | **823000 cP** |
| | | | | **88.9%** | **78.6%** | **77.2%** |
| | | | | | | |
| | **Stabilité chimique** | **Dosage (% actif relatif/ T0)** | **TA** | **101.4%** | **100.5%** | **100.0%** |
| | | | **40°C** | **101.2%** | **97.1%** | **99.1%** |

### Équipements utilisés:

Béchers formulaires de taille adaptée
Agitateur de type rotor-stator : Polytron®
Agitateur muni d'une pâle défloculeuse : Turbotest VMI-Rayneri

### Préparation des deux phases

Dans le bécher formulaire, peser tous les éléments de la phase grasse (A) et les chauffer à 70°C, sous agitation magnétique jusqu'à obtention d'un mélange homogène.

Dans un bécher annexe, peser tous les éléments de la phase aqueuse (B) et les chauffer à 70°C, sous agitation magnétique jusqu'à obtention d'un mélange limpide.

### Emulsification

Verser rapidement la phase aqueuse (B) sur la phase grasse (A) sous agitation forte : Rotor-stator : 10000 - 13000 rpm.

Maintenir l'agitation pendant 30 - 40 minutes, jusqu'à obtention d'une émulsion fluide fine.

### Refroidissement

Arrêter l'agitation au Polytron® et mettre le bécher contenant l'émulsion dans un bain-marie froid, pour favoriser un refroidissement rapide.

Mettre alors l'émulsion sous agitation lente (Pâle défloculeuse - 200 rpm).

Ajouter progressivement l'eau de refroidissement (phase C).

Maintenir l'agitation lente jusqu'à ce que la température de l'émulsion soit inférieure à 25°C.

### Gélification

Ajouter progressivement l'agent gélifiant (phase D) dans l'émulsion sous agitation modérée à forte :
Agitateur Rayneri avec pâle défloculeuse : 500 - 800 rpm.

Maintenir l'agitation jusqu'à obtention d'une crème lisse, brillante.

### Exemple 2: procédé de fabrication à l'échelle pilote (10 kg)

La composition qui suit est préparée selon le protocole indiqué ci-après. Les aspects macroscopique et microscopique à T0 sont étudiés.

Les résultats obtenus figurent à la suite de chaque composition.

| **EXEMPLE 2 - Composition** | | | | | |
|---|---|---|---|---|---|
| **Phase** | **Nom Commercial** | | **Nom INCI** | | **% (w/w)** |
| **A1** | **BRIJ S10-S0** | | **STEARETH-10** | | **1.25** |
| **A1** | **KOLLIWAX S** | | **STEARIC-/ PALMITIC ACID** | | **0.85** |
| **A1** | **BRIJ S2-SO** | | **STEARETH-2** | | **1.80** |
| **A1** | **KOLLIWAX SA** | | **STEARYL ALCOHOL** | | **0.25** |
| **A1** | **MIGLYOL 812 N** | | **CAPRYLIC/ CAPRIC TRIGLYCÉRIDES** | | **11.50** |
| **A1** | **ST-CYCLOMETHICONE 5-NF** | | **CYCLOPENTASILOXANE** | | **4.00** |
| **A2** | **PHENOXETOL** | | **PHENOXYETHANOL** | | **1.00** |
| **A2** | **MIGLYOL 812 N** | | **CAPRYLIC/ CAPRIC TRIGLYCÉRIDES** | | **4.00** |
| **A2** | **DL-ALPHA-TOCOPHÉROL** | | **ALPHA TOCOPHEROL** | | **0.20** |
| **B1** | **EAU PURIFIÉE** | | **AQUA (WATER)** | | **21.41** |
| **B1** | **GLYCÉRINE** | | **GLYCEROL** | | **5.00** |
| **B1** | **CHLORURE DE BENZALKONIUM** | | **BENZALKONIUM CHLORIDE** | | **0.08** |
| **B1** | **TRIÉTHANOLAMINE** | | **TRIETHANOLAMINE** | | **0.20** |
| **B2** | **EAU PURIFIÉE** | | **AQUA (WATER)** | | **41.06** |
| **B3** | **ALCOOL BENZYLIQUE** | | **BENZYL ALCOHOL** | | **0.40** |
| **B3** | **EAU PURIFIÉE** | | **AQUA (WATER)** | | **3.00** |
| **C** | **SIMULGEL 600 PHA** | | **ACRYLAMIDE / SODIUM ACRYLOYLDIMETHYL TAURATE COPOLYMER & ISOHEXADECANE & POLYSORBATE 80** | | **4.00** |
| | | | | | |
| **CARACTERISATION À T0** | | **ASPECT MACROSCOPIQUE** | | **Crème épaisse blanche brillante** | |
| | | **ASPECT MICROSCOPIQUE** | | **Tapis d'émulsion fin et homogène** | |
| | | **DIAMETRE MOYEN** | | **440 nm** | |

### Équipements utilisés:

Phase aqueuse : Fondoir OLSA
Phase grasse : Cuve OLSA (cuve de préparation)

### Préparation de la phase grasse (phases A1 + A2)

Dans une cuve OLSA, introduire sous agitation les différents éléments de la phase grasse A1. Agiter à l'aide de la turbine d'agitation à 1500 rpm.

Stopper la turbine d'agitation et chauffer le mélange à 70°C sous agitation coaxiale (30 rpm).

Ajouter le reste de la phase grasse (A2) sous agitation coaxiale jusqu'à obtention d'un mélange limpide.

### Préparation de la phase aqueuse (phase B1)

Dans un fondoir OLSA, introduire les différents éléments de la phase aqueuse (phase B1).

Sous agitation (150rpm), chauffer le mélange à 70°C en couvrant le fondoir pour limiter l'évaporation, jusqu'à ce que tous les excipients soient parfaitement solubilisés et que le mélange devienne limpide.

### Emulsification

Sous agitation turbine, transférer par aspiration fond de cuve très lentement la phase aqueuse (fondoir OLSA) dans la phase grasse (cuve OLSA) à l'aide d'un tuyau :
Vitesse coaxiale : 45 rpm
Vitesse turbine : 3000 rpm
Maintenir les agitations et la température (70°C) jusqu'à émulsification des deux phases et obtention d'un liquide blanc, opaque, brillant, présentant un tapis d'émulsion très fin au microscope optique.

### Refroidissement

Dans la cuve OLSA, introduire sous agitation, par aspiration fond de cuve l'eau de refroidissement (phase B2) :
Vitesse coaxiale : 45 rpm
Vitesse turbine : 3000 rpm
Refroidir le mélange à 40°C sous agitation :
Vitesse coaxiale : 45 rpm
Vitesse turbine : 3000 rpm
Lorsque le mélange est à 40°C, ajouter la phase B3 en maintenant l'agitation.

### Gélification

Introduire l'agent gélifiant (phase C) dans le mélange précédent à 40°C, sous agitation :
Vitesse coaxiale : 45 rpm
Vitesse turbine : 2000 rpm
Refroidir à 25°C sous agitation, et maintenir l'agitation jusqu'à obtention d'une crème blanche, brillante, lisse.

### Exemple 3: Procédé de fabrication à l'échelle industrielle (4000 kg)

La composition qui suit est préparée selon le protocole indiqué ci-après.

| **EXEMPLE 3 - Composition** | | | |
|---|---|---|---|
| **Phase** | **Nom commercial** | **Ingrédients - INCI** | **Teneur %** |
| **A** | **NEO HELIOPAN OS/BP** | **ETHYLHEXYL SALICYLATE OCTOCRYLENE** | **29.05** |
| | **NEO HELIOPAN 303** | | |
| | **PARSOL 1789** | **BUTYL METHOXYDIBENZOYLMETHANE** | |
| | **SILICONE FLUID 200 350cST** | **DIMETHICONE** | |
| | **TWEEN 61V** | **POLYSORBATE 61** | |
| | **AMISOFT HS11** | **SODIUM STEARYL GLUCAMATE** | |
| | **RYOTO SUGAR ESTER S370** | **SUCROSE TRISTEARATE** | |
| | **DUB DIS** | **DIISOPROPYLE SEBACATE** | |
| | **ELDEW SL 205** | **ISOPROPYL LAURYL SARCOSINATE** | |
| | **ENOXOLONE / PLANTACTIV GLA 18** | **GLYCYRRHETINIC ACID** | |
| **B** | **CÉRAMIDE 5** | **HYDROXYPALMITOYL SPHINGANINE** | **42.8** |
| | **EAU PURIFIÉE** | **AQUA (WATER)** | |
| | **RONACARE ALLANTOINE** | **ALLANTOIN** | |
| | **TITRIPLEX III** | **DISODIUM EDETATE** | |
| | **PHENOXETOL** | **PHENOXYETHANOL** | |
| | **HYDROLITE-5** | **PENTYLENE GLYCOL** | |
| | **DERMOSOFT OCTIOL** | **CAPRYLYL GLYCOL** | |
| | **D-PANTHENOL USP** | **PANTHENOL** | |
| | **GLYCERINE 4810** | **GLYCERIN** | |
| **C** | **DL ALPHA TOCOPHEROL ACETATE** | **TOCOPHERYL ACETATE** | **19.95** |
| | **EAU PURIFIÉE** | **AQUA (WATER)** | |
| | **-** | **SORBATE DE POTASSIUM** | |
| | **-** | **GLUCONATE DE ZINC** | |
| | **RHODICARE XT** | **XANTHAN GUM** | |
| | **CARBOPOL 980** | **CARBOMER** | |
| **D** | **TRIETHANOLAMINE CARE** | **TRIETHANOLAMINE** | **2.2** |
| | **DC 1503 FLUID** | **DIMETHICONE (AND) DIMETHICONOL** | |
| **E** | **DRY FLO PLUS** | **ALUMINIUM STARCH OCTENYLSUCCINATE** | **6** |
| | **SILICA BEAD SB 150** | **SILICA** | |
| | **MICROPEARL M100** | **POLYMETHYLMETHACRYLATE** | |

### Equipements utilisés :

# 4000 L skid avec fondoir (cuve de préparation)
2 contenants de 100 L en acier inoxydable
1 contenant de 200 L en acier inoxydable
2 contenants de 20 kg en acier inoxydable

### Préparation de la phase grasse (phases A)

Dans la cuve principale, introduire sous agitation les différents éléments de la phase grasse A.

Chauffer le mélange à 70°C sous agitation :
Vitesse coaxiale : 20 - 40 rpm
Râcleurs : 20 rpm
Vitesse turbine : 700 - 1500 rpm

### Préparation de la phase aqueuse (phases B)

Dans un fondoir, introduire les différents éléments de la phase aqueuse.

Chauffer le mélange à 70°C sous agitation :
Râcleurs : 10 - 20 rpm
Vitesse turbine : 1000 - 1500 rpm
Maintenir l'agitation et le chauffage, jusqu'à ce que tous les excipients soient parfaitement solubilisés et que le mélange devienne limpide.

### Emulsification

Sous agitation turbine, transférer par aspiration fond de cuve la phase aqueuse (fondoir) dans la phase grasse (cuve principale de préparation) à l'aide d'un tuyau :
Vitesse coaxiale : 60 - 100 rpm
Râcleurs : 25 rpm
Vitesse turbine : 1000 - 1800 rpm
Maintenir l'agitation jusqu'à émulsification des deux phases et obtention d'un liquide blanc, opaque, brillant, présentant un tapis d'émulsion très fin au microscope optique.

### Refroidissement

Refroidir le mélange de la cuve principale à 50°C sous agitation :
Vitesse coaxiale : 50 - 100 rpm
Vitesse turbine : 1000 - 1800 rpm

Peser dans un contenant approprié les éléments de la phase C, et agiter jusqu'à dispersion homogène des excipients dans l'eau :
Râcleurs : 20 - 30 rpm
Vitesse turbine : 2000 - 3000 rpm

Dans la cuve de préparation, introduire sous agitation, par aspiration fond de cuve la phase C de refroidissement :
Vitesse coaxiale : 40 rpm
Râcleurs : 20 rpm
Vitesse turbine : 1000 - 1800 rpm

### Gélification

Lorsque la préparation est à 40°C, introduire la phase D dans le mélange précédent sous agitation :
Râcleurs : 0 - 20 rpm
Vitesse turbine : 1000 - 1800 rpm

Ajouter les éléments de la phase E.

Maintenir l'agitation jusqu'à obtention d'une crème blanche, brillante, lisse.

Dans l'exemple 3, la teneur de la phase A est de 29.05% en poids, la teneur de la phase B est de 42,8% en poids, la teneur de la phase C est de 19,95% en poids, la teneur de la phase D est de 2,2% et la teneur de la phase E est de 6% en poids.

## Revendications

1. Procédé de fabrication d'une composition pharmaceutique ou cosmétique se trouvant sous la forme d'une émulsion de type huile-dans-eau formée de globules liquides pourvus chacun d'un enrobage cristal liquide lamellaire et dispersés dans une phase aqueuse, chaque globule liquide étant unitairement enrobé d'une couche monolamellaire ou oligolamellaire obtenue à partir d'au moins un agent tensioactif lipophile, d'au moins un agent tensioactif hydrophile et d'au moins un tensioactif anionique distinct ;
la composition étant apte à incorporer des excipients et/ou des principes actifs thermosensibles à des températures supérieures à 75°C,
ledit procédé comprenant les étapes suivantes :
a) mélanger à une température d'au plus 75°C l'agent tensioactif lipophile, l'agent tensioactif hydrophile, le tensioactif anionique distinct et tous les ingrédients des globules liquides, afin d'obtenir un mélange homogène de phase grasse (A),
b) mélanger à une température d'au plus 75°C les ingrédients de la phase aqueuse, afin d'obtenir un mélange homogène de phase aqueuse (B),
c) verser la phase aqueuse (B) obtenue en b) sur la phase grasse (A) obtenue en a) à l'aide d'un agitateur rotor-stator à une vitesse comprise entre 10000 rpm et 13000 rpm, et pendant un temps compris entre 25 minutes et 1 heure, afin d'obtenir une émulsion homogène,
d) refroidir l'émulsion homogène obtenue en c), en introduisant de l'eau dans le mélange obtenu en c), sous agitation à une vitesse comprise entre 1000 rpm et 3000 rpm, et à une vitesse coaxiale comprise entre 40 rpm et 50 rpm, jusqu'à ce que la température de l'émulsion soit inférieure ou égale à 50°C,
ledit procédé étant **caractérisé en ce que** le nombre d'étape est inférieur ou égal à 5, qu'il ne nécessite pas l'utilisation d'homogénéisateur haute pression, que ledit procédé ne présente pas d'étape de pré-émulsification et que toute étape dudit procédé est effectuée à une température inférieure ou égale à 75°C.

2. Procédé selon la revendication 1, **caractérisé en ce que** l'agent tensioactif lipophile présente une HLB comprise entre 2 et 5 et est choisi parmi le distéarate de sucrose, le tristéarate de sucrose, le distéarate de diglycéryle, le tristéarate de tétraglycéryle, le décastéarate de décaglycéryle, le monostéarate de diglycérol, le monostéarate de sorbitane, le tristéarate de sorbitane, le monostéarate de 15 diéthylène glycol, l'ester de glycérol et d'acides palmitique et stéarique, le monostéarate polyoxyéthyléné comportant 2 motifs oxyéthylène, le stéaryl éther polyoxyéthyléné comportant 2 motifs oxyéthylène, le mono- et dibéhénate de glycéryle et le tétrastéarate de pentaérythritol.

3. Procédé selon l'une des revendications 1 à 2, **caractérisé en ce que** l'agent tensioactif hydrophile présente une HLB comprise entre 8 et 12 et est choisi parmi le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le stéaryl éther polyoxyéthyléné 10 OE, le distéarate polyoxyéthyléné 12 OE et le distéarate de méthylglucose polyoxyéthyléné 20 OE.

4. Procédé selon l'une des revendications 1 à 3, **caractérisé en ce que** le tensioactif anionique distinct est choisi parmi les acides gras ou esters d'acide gras, ledit acide gras comprenant au moins une chaîne grasse saturée ayant plus de 12 atomes de carbone, de préférence entre 16 et 22, les lipides anioniques neutralisés, les lipides amphotères et les dérivés alkylsulfoniques.

5. Procédé selon l'une des revendications 1 à 4, **caractérisé en ce que** le tensioactif anionique distinct est choisi parmi l'acide stéarique, l'acide palmitique, l'acide arachidique, l'acide béhénique et le stéaroyl glutamate de sodium.

6. Procédé selon l'une des revendications 1 à 5, **caractérisé en ce que** les tensioactifs sont mélangés dans l'étape a) dans les proportions suivantes pour enrober chaque globule liquide :
∘ Tensioactif lipophile: 45-50% en poids du mélange ;
∘ Tensioactif hydrophile: 30-35% en poids du mélange ; et
∘ Tensioactif anionique distinct : 20-25% en poids du mélange.

7. Procédé selon l'une des revendications 1 à 6, **caractérisé en ce que** l'émulsion de type huile-dans-eau comprend au moins un principe actif, notamment choisi parmi les rétinoïdes, l'acide glycyrrhétinique et le gluconate de zinc, et de préférence l'acide 3"-tert-butyl-4'-(2-hydroxy-éthoxy)-4"-pyrrolidin-1-yl-[1,1';3',1"]-terphenyl-4-carboxylique.

8. Procédé selon l'une des revendications 1 à 7, **caractérisé en ce que** le mélange de l'étape a) se fait à l'aide d'une turbine d'agitation, de préférence à une vitesse comprise entre 700 rpm et 1500 rpm, et éventuellement avec une vitesse coaxiale comprise entre 20 rpm et 40 rpm.

9. Procédé selon l'une des revendications 1 à 8, **caractérisé en ce que** le mélange de l'étape b) se fait à l'aide d'une turbine d'agitation, de préférence à une vitesse comprise entre 150 rpm et 1500 rpm.

10. Procédé selon l'une des revendications 1 à 9, **caractérisé en ce que** le refroidissement de l'étape d) se fait par l'introduction d'eau dans le mélange obtenu en c), sous agitation à une vitesse comprise entre 1000 rpm et 3000 rpm, et à une vitesse coaxiale comprise entre 40 rpm et 50 rpm, jusqu'à ce que la température de l'émulsion soit inférieure ou égale à 40°C.

11. Procédé selon l'une des revendications 1 à 10, **caractérisé en ce qu'**un agent gélifiant est introduit dans le mélange refroidi obtenu en d), sous agitation à une vitesse comprise entre 1000 rpm et 2000 rpm, jusqu'à obtention d'une crème.

12. Utilisation du procédé selon l'une des revendications 1 à 11, pour la fabrication d'une composition comprenant au moins un excipient et/ou principe actif thermosensible se dégradant à des températures supérieures à 75°C.

## Patentansprüche

1. Verfahren zur Herstellung einer pharmazeutischen oder kosmetischen Zusammensetzung, welche sich in der Form einer Emulsion des Typs Öl-in-Wasser befindet, die aus flüssigen Kügelchen gebildet wird, jeweils versehen mit einer lamellaren Flüssigkristallbeschichtung und dispergiert in einer wässrigen Phase, wobei jedes flüssige Kügelchen einheitlich mit einer monolamellaren oder oligolamellaren Schicht beschichtet wird, die aus mindestens einem lipophilen grenzflächenaktiven Mittel, mindestens einem hydrophilen grenzflächenaktiven Mittel und mindestens einem anderen anionischen grenzflächenaktiven Mittel erhalten wird;
wobei die Zusammensetzung geeignet ist, Exzipienten und/oder hitzeempfindliche Wirkstoffe bei Temperaturen von mehr als 75°C einzuschließen;
wobei das Verfahren die folgenden Schritte umfasst:
a) Mischen, bei einer Temperatur von höchstens 75°C, des lipophilen grenzflächenaktiven Mittels, des hydrophilen grenzflächenaktiven Mittels, des anderen anionischen grenzflächenaktiven Mittels und aller Bestandteile der flüssigen Kügelchen, um eine homogene Mischung einer Fettphase (A) zu erhalten;
b) Mischen, bei einer Temperatur von höchstens 75°C, der Bestandteile der wässrigen Phase, um eine homogene Mischung der wässrigen Phase (B) zu erhalten;
c) Gießen der wässrigen Phase (B), die in b) erhalten wird, auf die Fettphase (A), die in a) erhalten wird, mit Hilfe eines Rotor-Stator-Rührers bei einer Geschwindigkeit zwischen 10000 UpM und 13000 UpM, und während einer Zeit zwischen 25 Minuten und 1 Stunde, um eine homogene Emulsion zu erhalten;
d) Abkühlen der homogenen Emulsion, die in c) erhalten wird, durch Einbringen von Wasser in die Mischung, die in c) erhalten wird, unter Rühren bei einer Geschwindigkeit zwischen 1000 UpM und 3000 UpM, und bei einer koaxialen Geschwindigkeit zwischen 40 UpM und 50 UpM, bis die Temperatur der Emulsion kleiner oder gleich 50°C ist;
wobei das Verfahren **dadurch gekennzeichnet ist, dass** die Anzahl von Schritten kleiner oder gleich 5 ist, wodurch keine Verwendung eines HochdruckHomogenisators notwendig ist, dadurch, dass das Verfahren keinen Voremulgierungsschritt aufweist, und dadurch, dass jeder Schritt des Verfahrens bei einer Temperatur kleiner oder gleich 75°C durchgeführt wird.

2. Verfahren nach Anspruch 1, **dadurch gekennzeichnet, dass** das lipophile grenzflächenaktive Mittel einen HLB zwischen 2 und 5 aufweist, und ausgewählt wird aus Saccharosedistearat, Saccharosetristearat, Diglyceryldistearat, Tetraglyceryltristearat, Decaglyceryldecastearat, Diglycerinmonostearat, Sorbitanmonostearat, Sorbitantristearat, 15-Diethylenglykolmonostearat, Glycerin- und Palmitin- und Stearinsäurestern, Polyoxyethylenmonostearat, umfassend zwei Oxyethylen-Einheiten, Polyoxyethylenstearylether, umfassend 2 Oxyethylen-Einheiten, Glyceryldibenat und Pentaerythrittetrastearat.

3. Verfahren nach einem der Ansprüche 1 bis 2, **dadurch gekennzeichnet, dass** das hydrophile grenzflächenaktive Mittel einen HLB zwischen 8 und 12 aufweist, und ausgewählt wird aus 4 OE Polyoxyethylensorbitanmonostearat, 20 OE Polyoxyethylensorbitantristearat, 8 OE Polyoxyethylenmonostearat, Hexaglycerylmonostearat, 10 OE Polyoxyethylenmonostearat, 10 OE Polyoxyethylenstearylether, 12 OE Polyoxyethylendistearat und 20 OE Polyoxyethylenmethylglucosedistearat.

4. Verfahren nach einem der Ansprüche 1 bis 3, **dadurch gekennzeichnet, dass** das andere anionische grenzflächenaktive Mittel ausgewählt wird aus Fettsäuren oder Fettsäureestern, wobei die Fettsäure mindestens eine gesättigte Fettkette mit mehr als 12 Kohlenstoffatomen, vorzugsweise zwischen 16 und 22, neutralisierte anionische Lipide, amphotere Lipide und Alkylsulfonderivate umfasst.

5. Verfahren nach einem der Ansprüche 1 bis 4, **dadurch gekennzeichnet, dass** das andere anionische grenzflächenaktive Mittel ausgewählt wird aus Stearinsäure, Palmitinsäure, Arachinsäure, Behensäure und Natriumstearoylglutamat.

6. Verfahren nach einem der Ansprüche 1 bis 5, **dadurch gekennzeichnet, dass** die grenzflächenaktiven Mittel in Schritt a) in den folgenden Anteilen gemischt werden, um jedes flüssige Kügelchen zu beschichten:
- lipophiles grenzflächenaktives Mittel: 45 bis 50 Gew.-% der Mischung;
- hydrophiles grenzflächenaktives Mittel: 30 bis 35 Gew.-% der Mischung; und
- anderes anionisches grenzflächenaktives Mittel: 20 bis 25 Gew.-% der Mischung.

7. Verfahren nach einem der Ansprüche 1 bis 6, **dadurch gekennzeichnet, dass** die Emulsion des Typs Öl-in-Wasser mindestens einen Wirkstoff umfasst, insbesondere ausgewählt aus Retinoiden, Glycyrrhetinsäure und Zinkgluconat, und vorzugsweise 3"-tert.Butyl-4'-(2-hydroxyethoxy)-4"-pyrrolidin-1-yl-[1,1';3',1"]-terphenyl-4-carbonsäure.

8. Verfahren nach einem der Ansprüche 1 bis 7, **dadurch gekennzeichnet, dass** das Mischen von Schritt a) mit Hilfe einer Rührturbine durchgeführt wird, vorzugsweise bei einer Geschwindigkeit zwischen 700 UpM und 1500 UpM, und gegebenenfalls mit einer koaxialen Geschwindigkeit zwischen 20 UpM und 40 UpM.

9. Verfahren nach einem der Ansprüche 1 bis 8, **dadurch gekennzeichnet, dass** das Mischen von Schritt b) mit Hilfe einer Rührturbine durchgeführt wird, vorzugsweise bei einer Geschwindigkeit zwischen 150 UpM und 1500 UpM.

10. Verfahren nach einem der Ansprüche 1 bis 9, **dadurch gekennzeichnet, dass** das Abkühlen von Schritt d) durch Einbringen von Wasser in die Mischung, die in c) erhalten wird, unter Rühren bei einer Geschwindigkeit zwischen 1000 UpM und 3000 UpM, und bei einer koaxialen Geschwindigkeit zwischen 40 UpM und 50 UpM durchgeführt wird, bis die Temperatur der Emulsion kleiner oder gleich 40°C ist.

11. Verfahren nach einem der Ansprüche 1 bis 10, **dadurch gekennzeichnet, dass** ein Gelierungsmittel in die abgekühlte Mischung, die in d) erhalten wird, unter Rühren bei einer Geschwindigkeit zwischen 1000 UpM und 2000 UpM eingebracht wird, bis eine Creme erhalten wird.

12. Verwendung des Verfahrens nach einem der Ansprüche 1 bis 11, zur Herstellung einer Zusammensetzung, umfassend mindestens einen Exzipienten und/oder hitzeempfindlichen Wirkstoff, der sich bei Temperaturen von mehr als 75°C zersetzt.

## Claims

1. Process for manufacturing a pharmaceutical or cosmetic composition in the form of an emulsion of oil-in-water type formed from liquid globules each bearing a lamellar liquid crystal coating and being dispersed in an aqueous phase, each liquid globule being individually coated with a monolamellar or oligolamellar layer obtained from at least one lipophilic surfactant, at least one hydrophilic surfactant and at least one distinct anionic surfactant;
the composition being able to incorporate excipients and/or active principles which are heat-sensitive to temperatures above 75°C,
said process comprising the following steps:
a) mixing, at a temperature not above 75°C, the lipophilic surfactant, the hydrophilic surfactant, the distinct anionic surfactant and all the ingredients of the liquid globules, so as to obtain a homogeneous mixture of fatty phase (A),
b) mixing, at a temperature not above 75°C, all the ingredients of the aqueous phase, so as to obtain a homogeneous mixture of aqueous phase (B),
c) pouring the aqueous phase (B) obtained in b) into the fatty phase (A) obtained in a) using a rotor-stator stirrer at a speed of between 10 000 rpm and 13 000 rpm, for a time of between 25 minutes and 1 hour, so as to obtain a homogeneous emulsion,
d) cooling the homogeneous emulsion obtained in c), by introducing water into the mixture obtained in c), with stirring at a speed of between 1000 rpm and 3000 rpm, and at a coaxial speed of between 40 rpm and 50 rpm, until the temperature of the emulsion is less than or equal to 50°C,
said process being **characterized in that** the number of steps is less than or equal to 5, **in that** it does not require the use of a highpressure homogenizer, **in that** said process does not involve a pre-emulsification step and **in that** every step of said process is performed at a temperature of less than or equal to 75°C.

2. Process according to Claim 1, **characterized in that** the lipophilic surfactant has an HLB of between 2 and 5 and is chosen from sucrose distearate, sucrose tristearate, diglyceryl distearate, tetraglyceryl tristearate, decaglyceryl decastearate, diglyceryl monostearate, sorbitan monostearate, sorbitan tristearate, 15 diethylene glycol monostearate, the glyceryl ester of palmitic and stearic acids, polyoxyethylene monostearate comprising 2 oxyethylene units, polyoxyethylene stearyl ether comprising 2 oxyethylene units, glyceryl monobehenate and dibehenate and pentaerythrityl tetrastearate.

3. Process according to either of Claims 1 and 2, **characterized in that** the hydrophilic surfactant has an HLB of between 8 and 12 and is chosen from polyoxyethylene sorbitan monostearate 4 OE, polyoxyethylene sorbitan tristearate 20 OE, polyoxyethylene monostearate 8 OE, hexaglyceryl monostearate, polyoxyethylene monostearate 10 OE, polyoxyethylene stearyl ether 10 OE, polyoxyethylene distearate 12 OE and polyoxyethylene methylglucose distearate 20 OE.

4. Process according to one of Claims 1 to 3, **characterized in that** the distinct anionic surfactant is chosen from fatty acids or fatty acid esters, said fatty acid comprising at least one saturated fatty chain containing more than 12 and preferably between 16 and 22 carbon atoms, neutralized anionic lipids, amphoteric lipids and alkylsulfonic derivatives.

5. Process according to one of Claims 1 to 4, **characterized in that** the distinct anionic surfactant is chosen from stearic acid, palmitic acid, arachidic acid, behenic acid and sodium stearoyl glutamate.

6. Process according to one of Claims 1 to 5, **characterized in that** the surfactants are mixed in step a) in the following proportions to coat each liquid globule:
∘ lipophilic surfactant: 45-50% by weight of the mixture;
∘ hydrophilic surfactant: 30-35% by weight of the mixture; and
∘ distinct anionic surfactant: 20-25% by weight of the mixture.

7. Process according to one of Claims 1 to 6, **characterized in that** the emulsion of oil-in-water type comprises at least one active agent, chosen especially from retinoids, glycyrrhetinic acid and zinc gluconate, and preferably 3"-tert-butyl-4'-(2-hydroxyethoxy)-4"-pyrrolidin-1-yl-[1,1';3',1"]-terphenyl-4-carboxylic acid.

8. Process according to one of Claims 1 to 7, **characterized in that** the mixing in step a) is performed using a turbomixer, preferably at a speed of between 700 rpm and 1500 rpm, and optionally with a coaxial speed of between 20 rpm and 40 rpm.

9. Process according to one of Claims 1 to 8, **characterized in that** the mixing in step b) is performed using a turbomixer, preferably at a speed of between 150 rpm and 1500 rpm.

10. Process according to one of Claims 1 to 9, **characterized in that** the cooling in step d) is performed by introducing water into the mixture obtained in c), with stirring at a speed of between 1000 rpm and 3000 rpm, and at a coaxial speed of between 40 rpm and 50 rpm, until the temperature of the emulsion is less than or equal to 40°C.

11. Process according to one of Claims 1 to 10, **characterized in that** a gelling agent is introduced into the cooled mixture obtained in d), with stirring at a speed of between 1000 rpm and 2000 rpm, until a cream is obtained.

12. Use of the process according to one of Claims 1 to 11, for the manufacture of a composition comprising at least one heat-sensitive excipient and/or active principle which degrade(s) at temperatures above 75°C.
